# EUROPEAN PATENT APPLICATION

(11) **EP 4 700 122 A1**
(43) Date of publication of application: **25.02.2026**
(21) Application number: 24191424.1
(22) Date of filing: 29.07.2024
(51) Int. Cl.: C12N 15/10

(54) **SAMPLE PREPARATION**

(71) Applicant: QU-IP B.V., 1015 CS Amsterdam (NL); UNIVERSITEIT TWENTE, 7522 NB Enschede (NL)
(72) Inventor: Verheijden, Mark, 3582 TJ Utrecht (NL); Aphrham, Samer, 1015 CS Amsterdam (NL); Huskens, Jurriaan, 7522 NB Enschede (NL); Das, Krishnendu, 7522 NB Enschede (NL)
(74) Representative: Arnold & Siedsma

(57) **Abstract**

The present invention relates to a method for isolating an electrically charged analyte from a body sample, comprising:
a) contacting the sample with solid particles coated with a molecule having an electric charge opposite to the electric charge of the analyte to allow the coating to capture the analyte by electrostatic interaction;
b) separating the solid particles and the sample; and
c) releasing the analyte from the solid particles by breaking down or destabilizing the coating.

When the coating is a polymer it is broken down or destabilized by means of an enzyme. When the coating comprises a metal ion that is chelated to the surface of the solid particles, the coating is destabilized or broken down by means of a reducing agent. The coating can also be hydrophobic comprising an amphiphilic molecule embedded therein and then the breaking down or destabilizing the coating is achieved by means of a detergent or a competitive agent.

## Description

The present invention relates to a method for purifying an analyte present in a body sample, in particular in a sample of a body fluid.

Biomolecules like proteins, carbohydrates, lipids, and nucleic acids, such as DNA and RNA, are commonly used as analytes in assays to understand biological processes, diagnose diseases, and study cellular components. ELISA, for example, detects proteins, antibodies, or antigens in samples. ELISA is widely used for diagnosing diseases (e.g., HIV), pregnancy tests, and measuring cytokines or soluble receptors. Nucleic acid assays, such as PCR, are used to detect DNA or RNA. Such assays are often performed on samples of body fluids or on biopsies from which the analyte is extracted by, for example, solubilizing and lysing the cells of the biopsy.

Sample preparation significantly influences the overall analytical process. Properly preparing a sample ensures accurate and reliable results in subsequent analyses. Body fluids, such as blood, urine, serum, saliva, tear fluid, etc., and biopsies, such as tissue samples, are rather complex and contain various components. Sample preparation removes interfering substances (e.g., proteins, lipids, salts) that can affect the measurement of the target analyte. By purifying the analyte from the interfering substances and/or increasing the concentration of the analyte, the accuracy and precision of the analysis improves.

A well-known technique for DNA extraction from a sample is based on the principle of ion exchange. Ion exchange relies on the interaction between positively-charged particles (ions) and the negatively-charged phosphates present in DNA. Some commercial ion-exchange formats exist in the market, for example from CD Bioparticles or Qiagen.

The Qiagen method is based on the interaction between negatively charged phosphates of the DNA backbone and positively charged diethylaminoethanol (DEAE) groups on the surface of a resin consisting of silica beads with a particle size of 100 µm, a large pore size, and a hydrophilic surface coating. Impurities such as RNA, protein, carbohydrates, and small metabolites are washed from the Qiagen resin with medium-salt buffers, while DNA remains bound until eluted with a high-salt buffer. The CD Bioparticle technology is based on magnetic polystyrene particles with polymeric polyethylenimine (PEI) coating.

The prior art processes have some disadvantages. Mainly, to elute the DNA from the beads, they generally make use of high salt conditions and/or high pH. This is not ideal or even fully incompatible with downstream analysis techniques (e.g. PCR, sequencing, biosensing) that are generally the aim of the sample preparation in the first place. As a result, downstream analyses require sample dilution or an additional (desalting) step, which are both disadvantageous.

It is therefore the object of the invention to provide an alternative analyte preparation method that avoids the above described drawbacks.

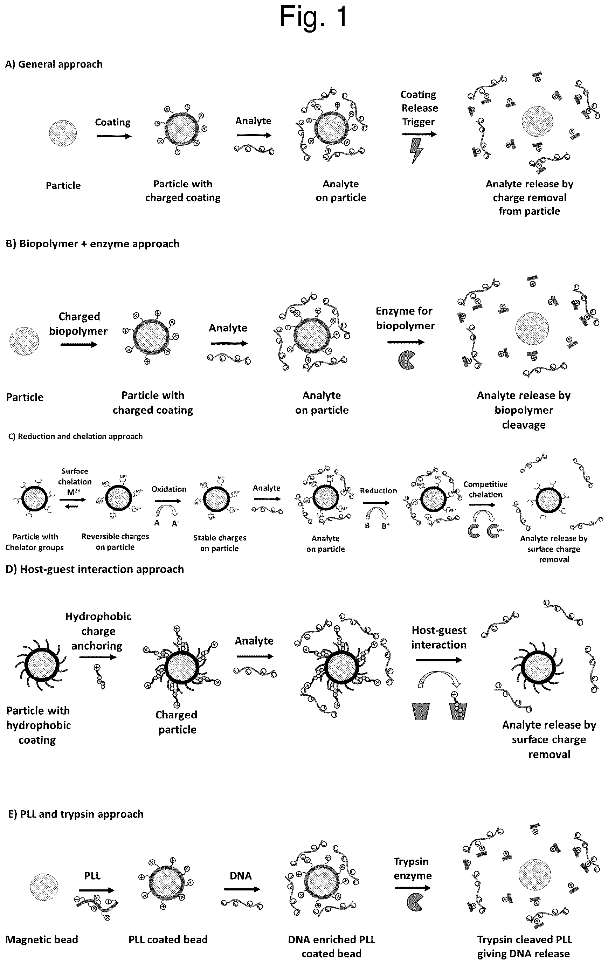

The present invention thus in general provides a method for isolating an electrically charged analyte from a sample of a body fluid, comprising:
a) contacting the sample with solid particles coated with a molecule having an electric charge opposite to the electric charge of the analyte to allow the coating to capture the analyte by electrostatic interaction;
b) separating the solid particles and the sample; and
c) releasing the analyte from the solid particles by breaking down or destabilizing the coating. This method is illustrated in **Figure 1A.** Breaking down or destabilizing the coating results in physical removal of the charge carriers from the particles' surface rather than using high salt or pH to overcome charge-charge interactions, which would have to be removed or adjusted before the sample could be used.

The type of coating and the mechanism of breaking down or destabilizing the coating may vary. According to one aspect of the invention, the coating is a polyelectrolyte polymer that is broken down or destabilized by means of an enzyme.

According to a further aspect, the coating comprises a metal ion that is in an oxidation state that results in kinetically trapped chelation to the surface of the solid particles. Addition of a reducing agent results in conversion of the metal ion to an oxidation state where it is more labile and hence easily removed by chelating molecules in solution.

According to a third aspect, the mechanism is based on host-guest chemistry wherein the coating is hydrophobic and comprise an amphiphilic molecule embedded therein and breaking down or destabilizing the coating is achieved by means of a detergent or a competitive agent. Host-guest chemistry involves the coordination of cationic, anionic or neutral guests within discrete host molecules or supramolecular host assemblies using noncovalent (H-bonding, ionic, van der Waals or hydrophobic) interactions.

In a particular embodiment, the method for isolating an electrically charged analyte from a sample of a body fluid comprises:
a) contacting the sample with solid particles coated with a polymer having an electric charge opposite to the electric charge of the analyte to allow the coating to capture the analyte by electrostatic interaction;
b) separating the solid particles and the sample; and
c) releasing the analyte from the solid particles by enzymatically digesting the polymer.
This method is illustrated in **Figure 1B.**

In a further embodiment, the method for isolating an electrically charged analyte from a sample of a body fluid comprises:
a) contacting the sample with solid particles coated with a chelating agent and a metal ion bound thereto, which metal ion has an electric charge opposite to the electric charge of the analyte to allow the coating to capture the analyte by electrostatic interaction;
b) separating the solid particles and the sample; and
c) releasing the analyte from the solid particles by reducing the metal ion and removing the reduced metal ions by chelating molecules in solution. This method is illustrated in **Figure 1C**
.

In another embodiment, the method for isolating an electrically charged analyte from a sample of a body fluid comprises:
a) contacting the sample with solid particles coated with a hydrophobic layer functionalized with an electrically charged amphiphilic molecule via a hydrophobic anchor, wherein the electric charge of the molecule is opposite to the electric charge of the analyte to allow the coating to capture the analyte by electrostatic interaction;
b) separating the solid particles and the sample; and
c) releasing the analyte from the solid particles by means of competition between the hydrophobic anchor and a competitor molecule, preferably added in solution. This method is illustrated in **Figure 1D**
.

.In all these embodiments, the solid particles are preferably magnetic beads that can be separated from the sample by means of magnetic interaction. Alternatively, the solid particles are comprised in a column and the sample is passed through the column.

Using this invention, the elution can be performed under mild conditions (e.g. low salt and neutral pH) since eluting the captured analyte from the beads is based on breaking down or destabilizing the coating, either by the action of an enzyme, a reducing agent optionally in combination with a chelator or a competitor molecule rather than elution with high salt concentrations. This allows direct downstream analysis without any intermediate steps or dilution, which saves both time and money. Furthermore, the risk of losing or creating a bias towards part of the analyte is reduced.

In one embodiment that is based on the enzymatic breakdown of the coating, the polymer is Poly-L-lysine (PLL). This method is illustrated in **Figure 1E** .At physiological pH, poly-L-lysine exhibits a high positive charge density which allows it to form complexes with negatively charged macromolecules, such as DNA or negatively charged proteins. To collect the analyte, poly-L-lysine is released from the magnetic beads by means of trypsin. Trypsin is a serine protease found in the digestive system of many vertebrates, where it hydrolyzes proteins. Trypsin cuts peptide chains mainly at the carboxyl side of the amino acids lysine or arginine. Upon contacting the beads with trypsin, the polymer bound to the beads, in particular magnetic beads, is broken down. This breakdown results in the polymer fragments detaching from the (magnetic) bead surface, together with the captured analyte. The analyte can then be used for further analysis.

Poly-L-lysine is particularly useful for negatively charged biomolecules, such as DNA and proteins. Other polymers, also with a positive charge or, alternatively with a negative charge, that can be used according to the invention include polymers with a negative charge, such as PLA (polylactic acid), which can be enzymatically cleaved e.g. with proteinase K, dextran, which can be enzymatically cleaved with dextranase, hyaluronic acid, which can be enzymatically cleaved with hyaluronidase or alginate, which can be enzymatically cleaved with alginate lyase.

Polymers with a positive charge are for example Poly-L-Arginine which can be enzymatically cleaved with trypsin, dextran functionalized with diethylaminoethanol (DEAE) groups and chitosan. DEAE functionalized dextran can be cleaved with dextranase. Chitosan can be cleaved with chitosanases. Several combinations of charged biopolymers and their degrading enzymes are listed in the table below.

| **Biopolymer** | **Charge** | **Degrading Enzyme** |
|---|---|---|
| Chitosan | Positive | Chitosanase |
| Poly-L-lysine | Positive | Trypsin |
| Gelatin | Positive | Gelatinase (MMP-2, MMP-9) |
| Poly-L-arginine | Positive | Trypsin |
| Protamine | Positive | Proteases |
| Eudragit E | Positive | Specific amidases/proteases |
| Polydopamine | Positive | Oxidative Enzymes |
| Spermidine | Positive | Amino Oxidases |
| Polyguanidine | Positive | Specific amidases/proteases |
| Poly-L-ornithine | Positive | Proteases (e.g., trypsin) |
| Polylactic acid | Negative | Proteinase K |
| Hyaluronic Acid | Negative | Hyaluronidase |
| Alginate/alginic acid | Negative | Alginate Lyase |
| Pectin | Negative | Pectinase |
| Heparin | Negative | Heparinase |
| Polyglutamic Acid | Negative | Glutaminase |
| Carboxymethylcellulose | Negative | Cellulase |
| Sialic Acid | Negative | Neuraminidase |
| Xylan | Negative | Xylanase |
| Chondroitin Sulfate | Negative | Chondroitinase |
| Dextran Sulfate | Negative | Dextranase |
| Poly(aspartic acid) | Negative | Aspartase |
| Carrageenan | Negative | Carrageenase |
| Gellan Gum | Negative | Gellan Lyase |

In a further embodiment, it is possible to make multilayers of charged polymers, for example first coating the beads with PLL and then a negative polymer. For removing the charged layers, trypsin can be used to cleave the supporting PLL layer.

Trypsin is a protease and can as such interfere in the downstream processing of the analyte. If the analyte is, for example, DNA intended for PCR then trypsin may affect the function of the PCR enzymes such as Taq DNA polymerase. In one embodiment, the method thus further comprises the step of adding an inhibitor of the enzyme.

In one embodiment, the enzyme is trypsin, and a trypsin inhibitor is used. A trypsin inhibitor is for example a type of serine protease inhibitor (serpin) that reduces the biological activity of trypsin by controlling the activation and catalytic reactions thereof. These are so-called suicide inhibitors that destructively alter trypsin thereby rendering it unavailable to bind with other proteins. By adding the inhibitor, trypsin is thus effectively inactivated and can no longer interfere with any of the analytical processes for which the biomolecule, in particular DNA, is used. Other protease inhibitors work by tightly binding to the active site of the enzyme, thus deactivating the enzyme.

Examples of suitable enzyme inhibitors are bovine pancreatic trypsin inhibitor (BPTI), which is a small, highly stable protein that binds very tightly to trypsin, blocking its active site, soybean trypsin inhibitor (STI), which is a large protein that can inhibit multiple types of proteases, including trypsin, and a human trypsin inhibitor, such as pancreatic secretory trypsin inhibitor, PSTI, a small protein that specifically inhibits trypsin and is also known as serine protease inhibitor Kazal-type 1 (SPINK1).

In one embodiment, the analyte is thus DNA. The method of the invention is, however, not limited thereto. The method of the invention can be used for any analyte selected from charged proteins, carbohydrates, lipids, and nucleic acids, such as DNA and RNA.

In one embodiment, the analyte is anionic DNA or RNA, and the polymer is a cationic polymer, in particular poly-L-Lysine (PLL). PLL is preferably digested by means of trypsin. The enzymatic activity of trypsin is abolished by adding a trypsin inhibitor to the purified analyte prior to further processing thereof.

The PLL coating route is very easy, fast and cheap. If the analyte is a nucleic acid, the nucleic acids ranging from very short (e.g. 50 base pairs) to very long (up to genomic DNA) can be captured and released.

In another embodiment, a multivalent and positively charged metal ion is chelated at the particle surface resulting in binding of negatively charged DNA. The metal ion can be easily removed from the surface using dissolved EDTA, which results in elution of DNA.

In one embodiment, nickel(II) is chelated at the particle surface, which results in binding of negatively charged DNA. The nickel can be easily removed from the surface using dissolved EDTA, which results in elution of DNA. See **Figure 1C**

.In one embodiment cobalt(II) is chelated at the particle surface. The Co(II) is then oxidized to Co(III) to create a much more stable as well as more positively charged complex. Oxidation is possible using various oxidizing agents such as hydrogen peroxide. This results in binding of negatively charged DNA. To achieve elution, the Co(III) can be reduced again to the Co(II) state which is easily removed from the surface using dissolved EDTA. This results in elution of the captured DNA. Reduction is possible using various reducing agents, such as sodium hydride or ascorbic acid.

In one embodiment, hydrophobically coated particles are functionalized with charge using cholesteryl-modified charged molecules. The cholesteryl will spontaneously partition into the hydrophobic coating and thus functions as an anchor for the charged molecule. The charges can be removed from the particle by the competitive host-guest interaction of the cholesteryl with a competitor molecule, such as beta-cyclodextrin. This will result in DNA elution.

The method of the invention does not require use of a column. The magnetic beads or particles can be added to the sample, stirred through it and after capturing the analyte be removed therefrom by means of a magnet. Particles that are not magnetic can for example be removed by centrifugation. The breakdown treatment is then performed on the recovered particles comprising the analyte. The analyte is subsequently released from the solid particles by means of the enzyme, reducing agent or competitive molecule and the solid particles are then removed from the sample, for example by means of a magnet or by centrifugation. In one embodiment, the inactivation of the enzyme is then performed on the remaining sample that contains the analyte and the enzyme. After this, the sample is ready for use in the desired assay.

The invention can also be performed in a column. The coated particles are packed in the column. Subsequently, the sample is passed though the column and the analyte captured by the charge on the coated particles. Then, the breaking down treatment is performed thus eluting the analyte from the column.

The invention thus relates to a process of purifying and concentrating positively or negatively charged biomolecules from body samples, such as body fluids or biopsies, using (magnetic) beads with a positively charged surface.

The present invention will be illustrated in the Examples that follow. In the Examples reference is made to the following figures:
**Figure 1****.** Schematic illustration of the approach for capturing and releasing charged biomolecules from body samples, such as urine, using various coatings and different mechanisms for removing the analyte from the particle surface.
**Figure 2****.** Coating, DNA binding and trypsin cleavage experimentally shown by fluorescence microscopy on microbeads.
**Figure 3****.** (left) Percentage of isolated (=captured) DNA as function of the bead concentration and (right) DNA recovery in elution step compared to spiked input DNA, as function of trypsin concentration
**Figure 4****.** Microscopic images of PLL-FITC coated particles.
**Figure 5****.** Microscopic analysis of trypsin mediated hydrolysis of PLL on the particle surface. Imaging was done at 40X magnification and 50ms exposure.
**Figure 6****.** Kinetics of trypsin mediated hydrolysis of PLL on the particle surface. Imaging was done at 40X magnification and 50ms exposure.
**Figure 7****.** Microscopic analysis of DNA binding on the PLL-coated particle surface.
**Figure 8****.** Trypsin mediated release of DNA from the particle surface.
**Figure 9****.** a) PLL-FITC coated particles, b) DNA coated particles.
**Figure 10****.** a) Experimental strategy to investigate the DNA isolation efficiency of the PLL-FITC coated particles. b) Calibration curve of DNA in Tris-EDTA buffer. c) Plot of DNA isolated as a function of PLL-FITC-coated particle concentration.
**Figure 11****.** a) Calibration curve of DNA in Tris-EDTA buffer. b) Plot of DNA isolated as a function of PLL-coated particle concentration.

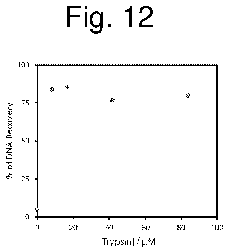

**Figure 12****.** DNA recovery as a function of Trypsin added.

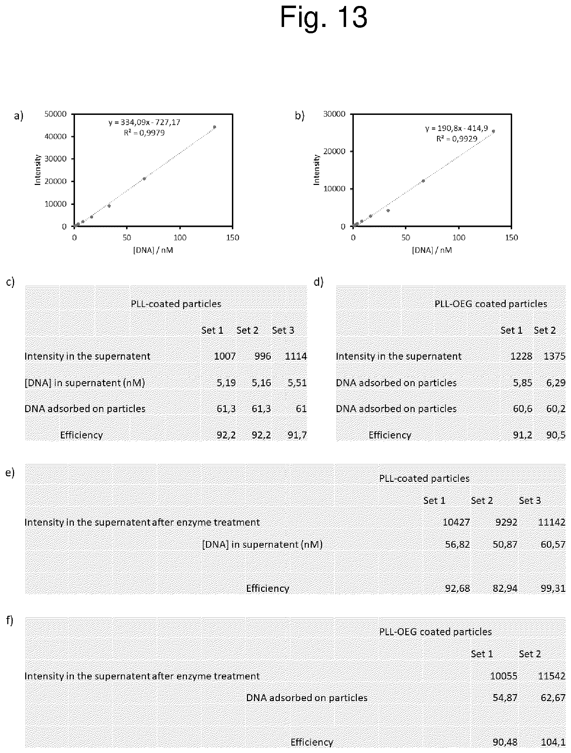

**Figure 13****.** a) Calibration curve of DNA in Sigmatrix. b) Calibration curve of DNA in Sigmatrix-Trypsin (42 uM). c) DNA isolation efficiency of PLL-coated magnetic particles, [Particle] = 0.4 mg/mL; [DNA] = 66.5 nM. d) DNA isolation efficiency of PLL-OEG-coated magnetic particles, [Particle] = 0.4 mg/mL; [DNA] = 66.5 nM. e) Recovery of DNA using PLL-coated particles in presence of 42 uM trypsin. f) Recovery of DNA using PLL-OEG-coated particles in presence of 42 uM trypsin.

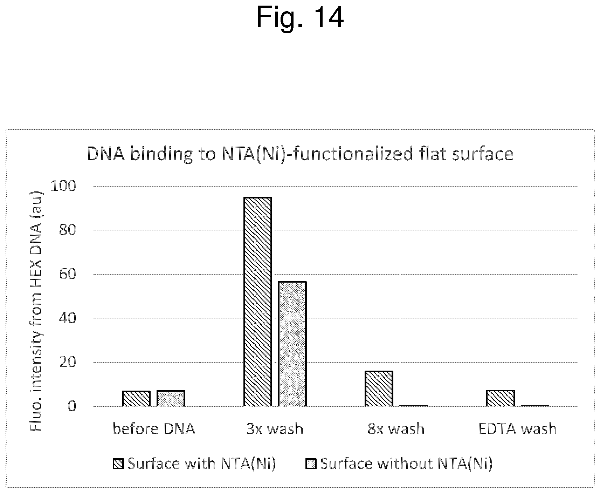

**Figure 14****.** Alternative route for creating charged surfaces where the charge carrier can be physically removed from the surface. Here, a flat surface was coated with a supported lipid bilayer (SLB) made up from dipalmitoylphosphatidylcholine (DOPC) phospholipids. A 100% DOPC control layer was compared with a 99% DOPC layer with 1mol% lipids containing chelated nickel(II), 18:1 DGS-NTA(Ni). Washing with EDTA to remove the surface bound nickel(II) resulted in full DNA elution.

### EXAMPLES

### EXAMPLE 1

### Testing the method steps

### Introduction

Poly-L-lysine (PLL) is a cationic polymer at physiological pH whereas DNA is an anionic biopolymer. The inventors anticipated that upon mixing them together, DNA would get bound on the PLL. Therefore, magnetic beads were coated with PLL, as macroscopic beads can be easily isolated from the solutions using an external magnet. In the next step, these PLL coated cationic magnetic beads were mixed with a DNA containing aqueous solution, so that all DNA gets bound on the magnetic bead surface through electrostatic interaction. The DNA-adsorbed magnetic beads were separated from the solution under external magnetic influence. In the next step, the DNA loaded magnetic beads were redisperse in an enzyme (Trypsin) solution to release the DNA from the bead surface. Trypsin is a well-known enzyme for digesting PLL and converting it to small fragments. Consequently, no driving force remains for DNA to remain bound on the bead surface and thereby, DNA gets released in the solution.

In this example the following three key steps are explored:
- Successful coating of particles with PLL
- Trapping DNA with PLL-coated particles through electrostatic interactions
- Trypsin mediated hydrolysis of PLL to release the DNA from the particle surface

### Materials and methods

### Step 1: Coating of the particle surface with Poly-L-lysine (PLL):

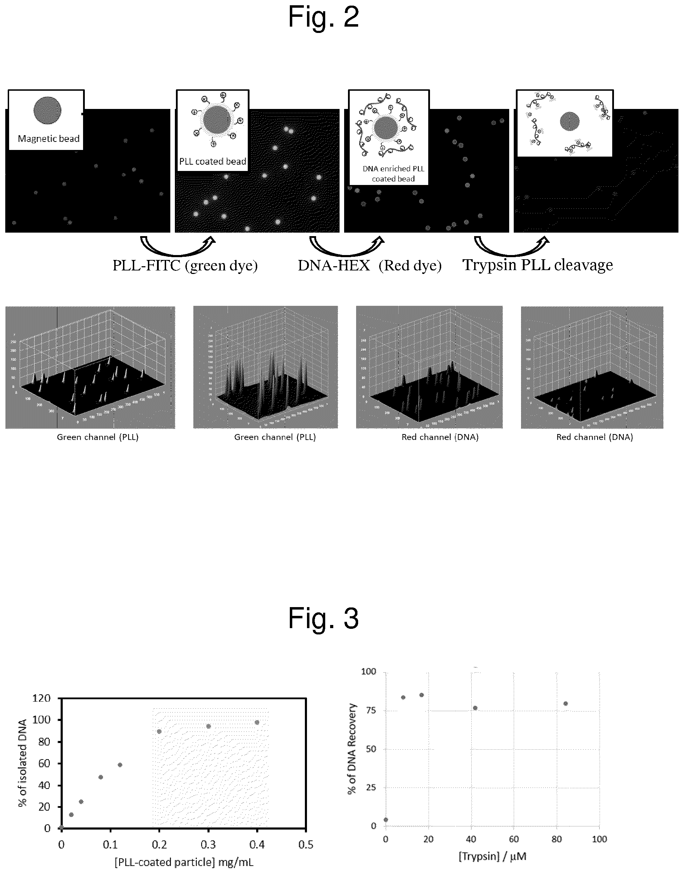

In the first experiment porous silica microparticles were coated with FITC functionalized PLL of Sigma Aldrich (Molecular Weight: 15000 - 30000; extent of coating: 0.3-1%: 0.003-0.01 mol FITC per mol lysine monomer) to be able to visualize the coating. The microparticles used were DiagNano Green Fluorescent Silica Particles of CD Bioparticles (DNG-L034; about 5 mm diameter; excitation at 485 nm; emission at 510 nm) in a concentration of 50 mg/mL in water. These are non-magnetic beads with larger size than the final format. FITC functionalized PLL was used for monitoring the surface coating under microscope, see **Figure 2****.**

13.6 µl of the 10 wt% stock suspension of beads was incubated for 1 h in a 2% Hellmanex solution on a rotary bench. These beads were centrifuged (500 rcf,; 5 min) and washed 3x with 2 ml Milli-Q water and finally suspended in 1 ml Milli-Q water and vortexed briefly to make the solution homogeneous. 100 µl of this suspension was added to 900 µl of PLL-FITC solution (0.1 mg/mL). Surface coating was allowed to take place for 1 h on the rotary bench. After the coating is done, excess PLL-FITC was removed by gentle centrifugation at 100 rcf for 5 min. The supernatant was replaced with 2 ml Milli-Q water and the process was repeated 2 more times and briefly vortexed after the last washing step. At the end 1 mL PBS (pH 8.0, 10 mM) buffer was added and the solution was used for further experiments.

The particles were imaged using fluorescence microscopy. The presence of brightly green, fluorescent particles confirmed the successful coating of the particles. The particles are slightly auto-fluorescent, but after imaging under the same exposure, it was clear that PLL-FTIC coated particles are much brighter (and the dots larger). It indicates the successful coating of the microparticles with PLL-FITC. This is illustrated in **Figure 2****.**

Next, the trypsin mediated hydrolysis of PLL-FITC on the particle surface was tested.

A stock solution of trypsin from porcine pancreas was prepared by dissolving 8 mg of trypsin in 4 mM HCL (pH 2.0). The stock solution concentration was then 336 mM.

In the next step, it was investigated whether trypsin can hydrolyze PLL on the particle surface. Trypsin is well known for hydrolyzing amide bonds in the vicinity of lysine. Therefore, PLL-FTTC coated particles (90.67 µg/mL) were incubated with 112 mM trypsin (from porcine pancreas) for 20 min and then again visualized under the microscope. After incubation with trypsin for 20 min., the particles were much less bright, and it could also be noticed that the background was becoming more greenish **(****Figure 2**). This indicates the hydrolysis of PLL-FITC and removal from the surface. But in case of uncoated particles, before and after incubation with trypsin, nothing changes **(****Figure 3**).

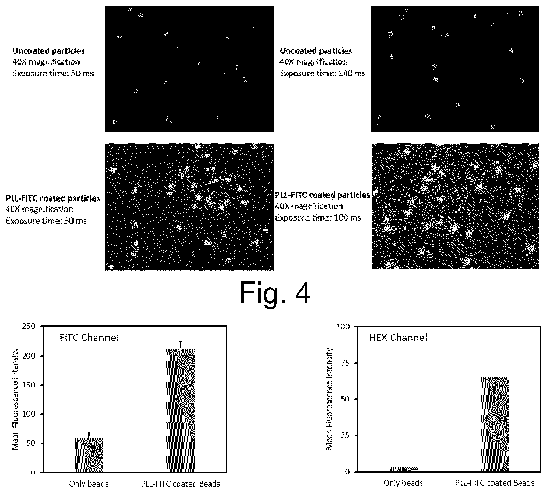

In the next step a hydrolysis kinetics was executed. PLL_FITC coated particles (90.67 µg/mL) were incubated with 112 mM trypsin (from porcine pancreas) and the particles monitored under the microscope. **Figure 4** clearly shows the decrease in the particle fluorescence and increase in background fluorescence over time.

### Step 2: Capturing DNA on the PLL-coated Silica Beads:

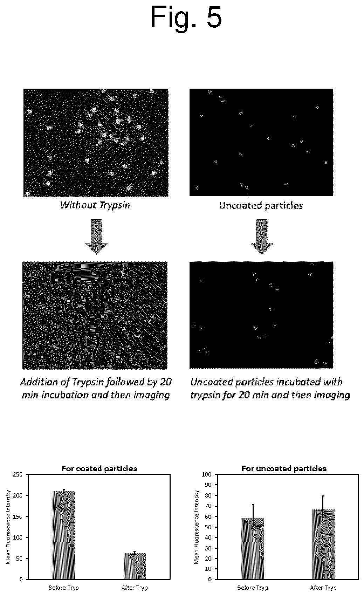

Then, the DNA binding was explored with these PLL-coated particles. PLL-coated particles (136 µg/mL) were incubated with 0.4 mM solution of DNA tagged with a red fluorescent dye called HEX, for 30 min. After that the particles were washed 3 times with PBS buffer to remove the excess DNA from the system. After that, the particles were observed under the microscope. The presence of bright red fluorescent particles confirmed the binding of DNA on the PLL-coated particle surface **(****Figure 5**).

### Step 3: Trypsin mediated hydrolysis of PLL followed by DNA release

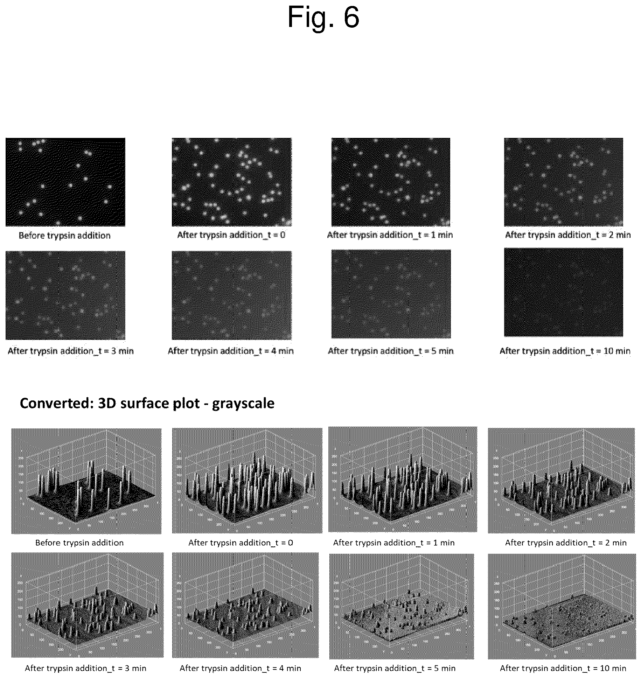

The DNA release from the surface upon trypsin mediated hydrolysis of PLL was investigated. The DNA coated particles (162 µg/mL) were incubated with 62 µM trypsin for 20 min. and then the particles were observed under the microscope. Microscope images showed sharp decrease in the red fluorescence from the particle surface which clearly indicates the trypsin mediated hydrolysis of PLL releases DNA within the solution (**Figure 6**).

### EXAMPLE 2

### Coating Magnetic Particles with PLL-FITC followed by DNA Capture

### Materials and methods

MagSi-DNA 3.0 COOH particles (Magtivio) with a diameter of 3.0 µm were used in a stock concentration of 20 mg/mL.

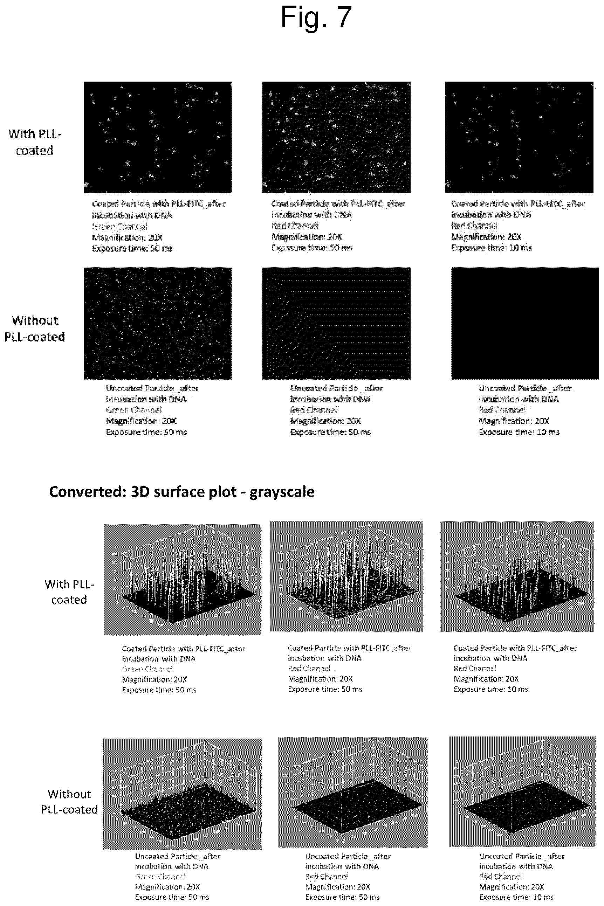

For coating the particles, 40 µL of the stock solution (20 mg/mL) was incubated with Hellmanex (2%) solution for 30 min. Then the particles were washed with MilliQ water 3 times followed by buffer (Tris-EDTA, pH 8.0, 10 mM) 2 times and finally redissolved in 1 mL of PLL-FITC solution (0.1 mg/mL) and incubated for 30 min. After the incubation period, the magnetic beads were washed with buffer for 3 times and redispersed in 1 mL buffer. Microscopic analysis clearly shows the successful coating of the particles with PLL (**Figure 7a**).

10 µL of the PLL-FITC coated particles (0.8 mg/mL stock solution) was incubated with 100 µL of DNA solution (133 nM stock solution). The final volume was adjusted with buffer to 200 µL and left for incubation for 30 min. Then the particles were washed 3 times with buffer and redispersed in 200 µL of buffer followed by imaging.

In 200 µL, the final concentration of DNA is 66.5 nM and of the coated particles it is 0.04 mg/mL.

Microscopic analysis revealed that these PLL coated magnetic particles are efficient to trap DNA on the surface (**Figure 7b**).

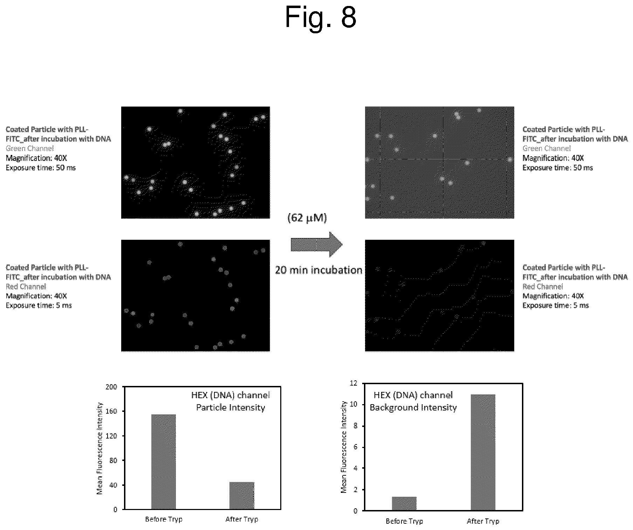

In the next step, a fixed concentration of DNA (133 nM) was incubated with different concentration of PLL-FITC coated particles for 30 min and then the particles were separated using a magnet. The fluorescence intensity of the supernatant was measured to quantify the unbound concentration of the DNA and subsequently the amount of DNA separated by PLL coated particles was calculated. A standard curve was used to quantify the DNA concentration using a fluorescence plate reader. At 0.2 mg/mL particle concentration, the maximum DNA was isolated (**Figure 8**) for this DNA concentration and incubation time. Importantly, it was found that almost 100% of the spiked DNA was captured.

### EXAMPLE 3

### Coating Magnetic Particles with PLL followed by DNA Capture

In this example, the knowledge from the PLL-FITC system was transferred to the PLL system only, since the FITC bound to the PLL was only used to follow and optimize the PLL coating route and is not needed in the final system.

40 µL of the stock solution (20 mg/mL) was incubated with Hellmanex (2%) solution for 30 min. Then the particles were washed with MilliQ water 3 times followed by buffer (Tris-EDTA, pH 8.0, 10 mM) 2 times and finally redissolved in 1 mL of PLL solution (0.1 mg/mL) and incubated for 30 min. After the incubation period, the magnetic beads were washed with buffer for 3 times and redispersed in 1 mL buffer.

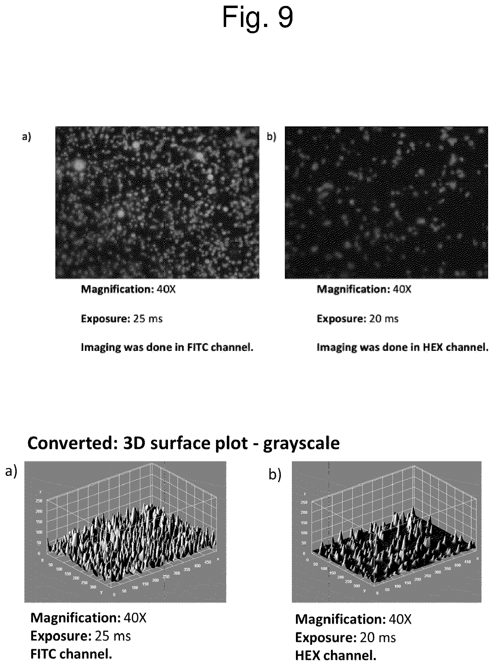

In the next step, a fixed concentration of DNA (133 nM) was incubated with different concentrations of PLL coated particles for 30 min and then the particles were separated using a magnet. The fluorescence intensity of the supernatant was measured to quantify the unbound concentration of the DNA and subsequently the amount of DNA separated by PLL coated particles was calculated. A standard curve was used to quantify the DNA concentration using a fluorimeter. It was found that at 0.2 mg/mL particle concentration, maximum DNA was isolated (**Figure 9**).

In the next step, the entire DNA capture to release together with PLL-coated particles was studied. For that purpose, 66.5 nM DNA was incubated with 0.4 mg/mL PLL-coated particles in 4 independent samples for 30 minutes (see above for details of the protocol). Then, the adsorbed DNA concentration on the particle surface was measured as stated above. After that, the magnetically isolated particles were redispersed in Tris-EDTA buffer containing different concentrations of Trypsin for 30 min. Thereafter, the particles were again pulled down with a magnet and the fluorescence intensity of the supernatant was measured to find out the concentration of the released DNA. The DNA loading study was done by using a calibration curve in Tris-EDTA buffer and the release study was done by using a calibration curve done in Tris-EDTA-Trypsin (84 µM) solution. It was observed that in the measured range of trypsin (8.4-84 um) the release occurred almost completely, independently of the concentration.

### EXAMPLE 4

### Isolation of DNA from Sigmatrix (synthetic urine)

In order to more closely investigate the compatibility of the protocol with the application of DNA capture in urine, the same protocol was followed but with a synthetic urine model matrix called Sigmatrix (Sigma-Aldrich), instead of Tris-EDTA buffer.

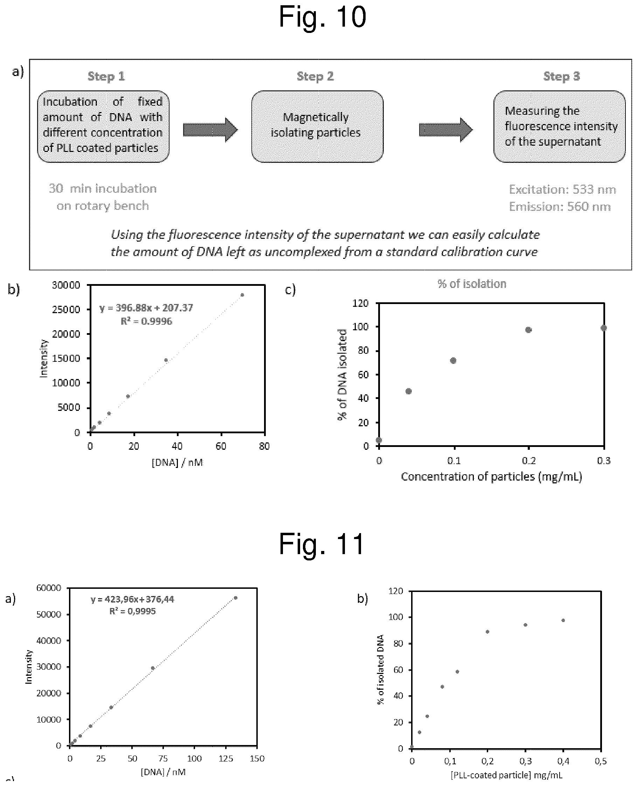

Here it was observed that in the presence of PLL-coated particles (0.4 mg/mL) the DNA adsorption efficiency is around: 91-92% (**Figure 11c**). The recovery of DNA using trypsin ranged from 82-99%.

This was tested for PLL and PLL with a fraction of the lysine residues functionalized with oligoethylene glycol OEG. This was done to stabilize the particles (prevent aggregation) in the more complex urine-like matrix. It did not seem to have an effect on the extraction or elution.

## Claims

1. Method for isolating an electrically charged analyte from a body sample, comprising:
a) contacting the sample with solid particles coated with a molecule having an electric charge opposite to the electric charge of the analyte to allow the coating to capture the analyte by electrostatic interaction;
b) separating the solid particles and the sample; and
c) releasing the analyte from the solid particles by breaking down or destabilizing the coating.

2. Method as claimed in claim 1, wherein the coating is a polymer that is broken down or destabilized by means of an enzyme.

3. Method as claimed in claim 1, wherein the coating comprises a metal ion that is chelated to the surface of the solid particles, and wherein the coating is destabilized or broken down by means of a reducing agent.

4. Method as claimed in claim 1, wherein the coating is hydrophobic and comprises an amphiphilic molecule embedded therein and the breaking down or destabilizing the coating is achieved by means of a detergent or a competitive agent.

5. Method as claimed in claim 1 and 2, comprising:
a) contacting the sample with solid particles coated with a polymer having an electric charge opposite to the electric charge of the analyte to allow the coating to capture the analyte by electrostatic interaction;
b) separating the solid particles and the sample; and
c) releasing the analyte from the solid particles by enzymatically digesting the polymer.

6. Method as claimed in claim 5, further comprising the step of adding an inhibitor of the enzyme.

7. Method as claimed in claim 1 and 3, comprising:
a) contacting the sample with solid particles coated with a chelating agent and a metal ion bound thereto, which metal ion has an electric charge opposite to the electric charge of the analyte to allow the coating to capture the analyte by electrostatic interaction;
b) separating the solid particles and the sample; and
c) releasing the analyte from the solid particles by reducing the metal ion and optionally chelating the reduced ion using a dissolved chelator agent.

8. Method as claimed in claim 1 and 4, comprising:
a) contacting the sample with solid particles coated with a hydrophobic layer functionalized with an electrically charged molecule via an amphiphilic anchor, wherein the electric charge of the molecule is opposite to the electric charge of the analyte to allow the coating to capture the analyte by electrostatic interaction;
b) separating the solid particles and the sample; and
c) releasing the analyte from the solid particles by means of competition between the amphiphilic anchor and a competitor molecule.

9. Method as claimed in any one of the claims 1 to 8, wherein the solid particles are magnetic beads that can be separated from the sample by means of magnetic interaction.

10. The method as claimed in any one of the claims 1-5 and 7-9, wherein the analyte is selected from charged proteins, carbohydrates, lipids, and nucleic acids.

11. The method as claimed in any one of the claims 1, 2 and 5, wherein the analyte is anionic DNA or RNA and the polymer is a cationic polymer.

12. The method as claimed in claim 1, 2, 5 and 11, wherein the polymer is Poly-L-lysine (PLL).

13. The method as claimed in claim 12, wherein Poly-L-lysine is released from the magnetic beads by means of trypsin.

14. The method as claimed in claim 13, wherein trypsin is deactivated by means of a trypsin inhibitor.
